# EUROPEAN PATENT APPLICATION

(11) **EP 2 774 921 A1**
(43) Date of publication of application: **10.09.2014**
(21) Application number: 14158096.9
(22) Date of filing: 06.03.2014
(51) Int. Cl.: C07D 251/54, A61Q 17/04

(54) **Crystalline form of a triazine derivative, the procedure for its production and cosmetic formulations thereof**

(30) Priority: 08.03.2013 IT MI20130357
(71) Applicant: 3V SIGMA S.p.A, 20121 Milano (IT)
(72) Inventor: Berte', Ferruccio, 24100 Bergamo (IT); Fabbi, Massimo, 24100 Bergamo (IT); Bemporad, Luca, 24100 Bergamo (IT); Maestri, Francesco, 24100 Bergamo (IT)
(74) Representative: Minoja, Fabrizio

(57) **Abstract**

Disclosed is a crystalline form of the compound of formula (I), characterised by an endothermic melting transition between 20 and 30 J/g at temperatures from 125°C to 135°C, determined by differential scanning calorimetry (DSC) and by its X-ray diffraction spectrum.

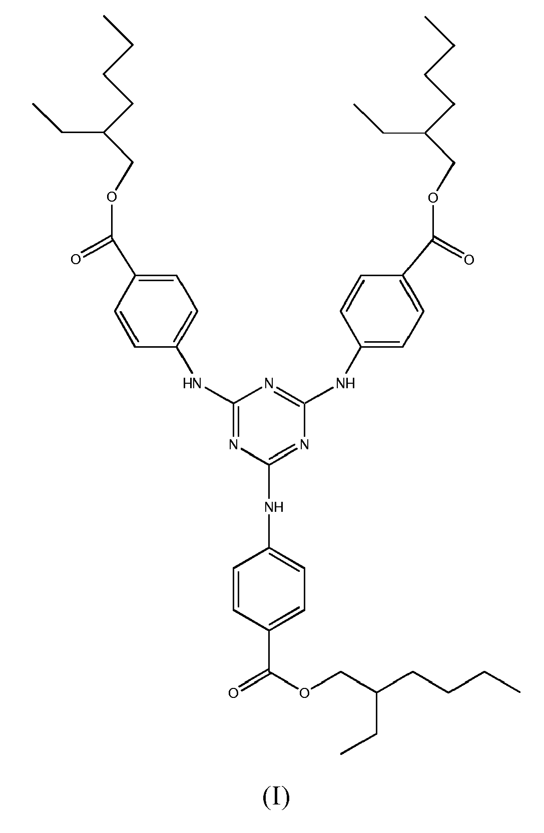

## Description

The present invention relates to a crystalline form of an s-triazine derivative, the process for the preparation thereof and the use thereof as a sunscreen or light stabiliser.

### Prior art

Ultraviolet solar radiation has a damaging effect on the skin tissue, and causes the degradation of polymers. By using particular compounds, called sunscreens, which absorb the UV part of solar radiation, harmful effects and aging of the skin and polymer materials can be prevented, or at least slowed.

A number of products have been studied and tested as protective agents, and a great deal of patent literature now exists relating to compounds belonging to various chemical classes that absorb in the ultraviolet region, particularly radiation between 290 and 320 nm, called UV-B, which is very harmful.

Relatively few of these compounds have proved suitable for practical application. They include p-methoxycinnamic acid and p-dimethylaminobenzoic acid esters, benzotriazoles and hydroxybenzophenones.

A drawback shared by all these compounds is their low ability to absorb radiation between 290 and 320 nm, which means that relatively large amounts are required to obtain the optimum photoprotective effect.

An excellent UV-B absorber should have the following characteristics:
1) High specific extinction at 290-320 nm allowing the use of low doses, resulting in cost savings and minimal toxicological risk
2) Light stability
3) Heat stability
4) Oxidation stability
5) Stability to different pHs
6) Good solubility in the basic substances commonly used for dermatological formulations
7) Negligible toxicity
8) Colour and odour compatible with the intended applications
9) High molecular weight, which reduces the probability of absorption by the skin and increases toxicological safety
10) Compatibility with the different substances generally used in dermatological formulations.

US 4617390 and US 4724137 disclose s-triazine derivatives obtained by reacting trichlorotriazine with p-amino-benzoic acid esters, which absorb intensely in the UV-B zone. Unfortunately, the solubility of these compounds in the solvents generally used to formulate sun creams is very low, which makes their practical use problematic and very difficult, especially when the percentage of photoprotector in the composition must be increased to prepare formulations with a high sun protection factor.

One of these compounds in particular, namely 2,4,6-trianilino-p-(carbo-1'-ethylhexyl-1'-oxy)-1,3,5-triazine, also known as octyl triazone, is widely used and has obtained authorisations on the market as a photoprotector of formulations and the skin in the field of cosmetic sunscreens. This compound, which is identified by the CAS number 88122-99-0, has the INCI name ethylhexyl triazone, and is known on the market under various names, including Uvinul T 150 (Basf) and Uvasorb ET (3V Sigma Spa), has the following structure.

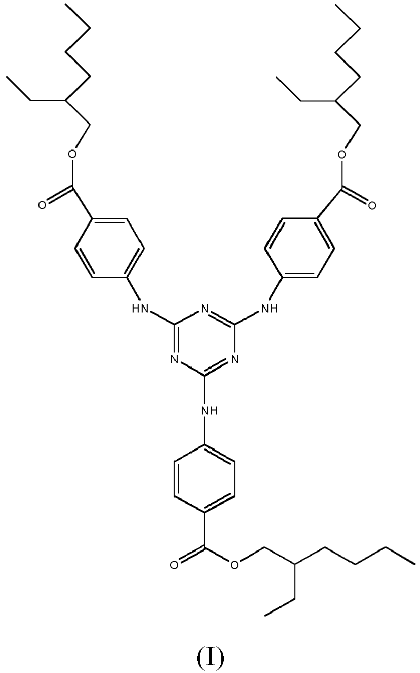

Over the years, some attempts have been made to improve the solubility of said compound in the solvents and oils generally used in cosmetic formulations.

US4656272 discloses its preparation in the presence of esters of branched alkanoic acids, and its isolation in mixtures with them.

US6531117 discloses stable aqueous dispersions of various sunscreens including stable aqueous colloidal dispersions of said compound in its amorphous or partly amorphous form, divided at microscopic level and obtained by particular precipitation techniques from solvents in the presence of protective colloids.

US7074922 discloses the preparation of a tautomeric form of compound I by a particular process of recrystallisation of its tautomeric forms from particular mixtures of solvents.

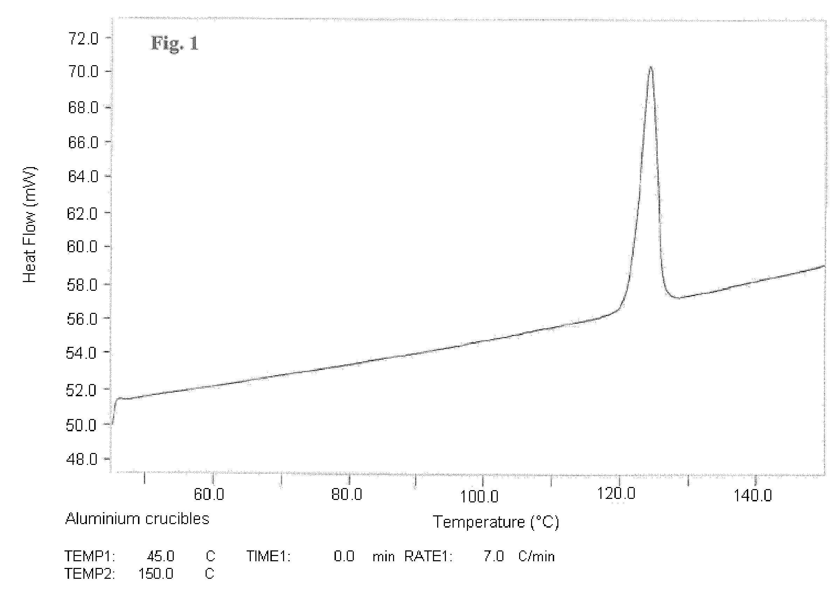

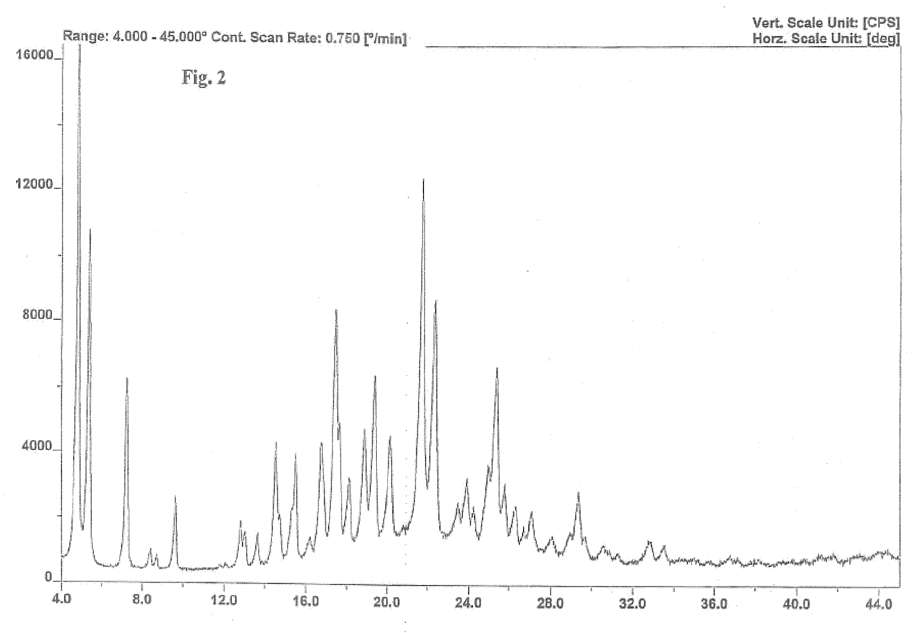

The crystalline form already known and currently on the market under the names Uvinul T150 (Basf) and Uvasorb ET (3V Sigma) is described in said patents US 4617390 and US 4724137. In particular, said crystalline form is obtainable by recrystallisation from solvents and mixtures thereof and subsequent drying. Its capillary melting point is about 128°C. However, the differential scanning calorimetry technique (DSC) shows in correspondence a very intense endothermic transition with a peak at 126°C. The energy contribution of this transition is 50-60 J/g of substance. A typical DSC scan of this product is reported in Figure 1. The X-ray powder diffraction spectrum of this crystalline form is shown in Figure 2. It is not readily soluble in the cosmetic oils used in sunscreen formulas designed to protect the skin, such as caprylic/capric triglyceride.

### Description of the invention

A crystalline form of the compound of formula (I) with advantageous characteristics has now been found.

The crystalline form of the invention can be obtained by treatment at temperatures exceeding 30°C, and in any event lower than or equal to the melting point of an amorphous form of the compound of formula (I), preferably of the vitreous amorphous solid form characterised by an exothermic transition at temperatures lower than 125°C, determined by differential scanning calorimetry (DSC). Said preferred amorphous form, disclosed in Italian patent application MI2012A002009 of 27.11.2012, is obtainable by rapid cooling of molten compound (I) on a cold surface or in a cold fluid, or by rapid elimination of the solvent from a solution of compound (I) at temperatures lower than its melting point.

The crystalline form of the invention can also be obtained by slow, controlled cooling of the molten mass of compound (I) at temperatures equal to or lower than its melting point or by treatment of the known crystalline form at temperatures exceeding 120°C and lower than its melting point.

Alternatively, the crystalline form of the invention can be obtained by removing the solvent from a solution of the compound of formula (I) at temperatures lower than its melting point.

The crystalline form of the invention is stable at normal storage temperatures for long periods, and can easily be ground to form a powder that is not caked, and is easy to use.

The novel crystalline form in powder, granule or flake form is far more rapidly solubilisable in common solvents and cosmetic oils than the corresponding known crystalline form. Moreover, unlike the known crystalline form, the crystalline form of the invention is soluble in shorter times in cosmetic oils at much lower temperatures, and even at room temperature.

The crystalline form of the invention is easily identifiable by thermal analysis techniques (such as DSC) and X-ray diffractometry.

Said crystalline form is characterised by an endothermic melting transition at temperatures from 125°C to 135°C with enthalpy between 20 and 30 J/g, very different from the fusion enthalpy of the known crystalline form, between 50 and 60 J/g and detectable at temperatures from 120°C to 130°C.

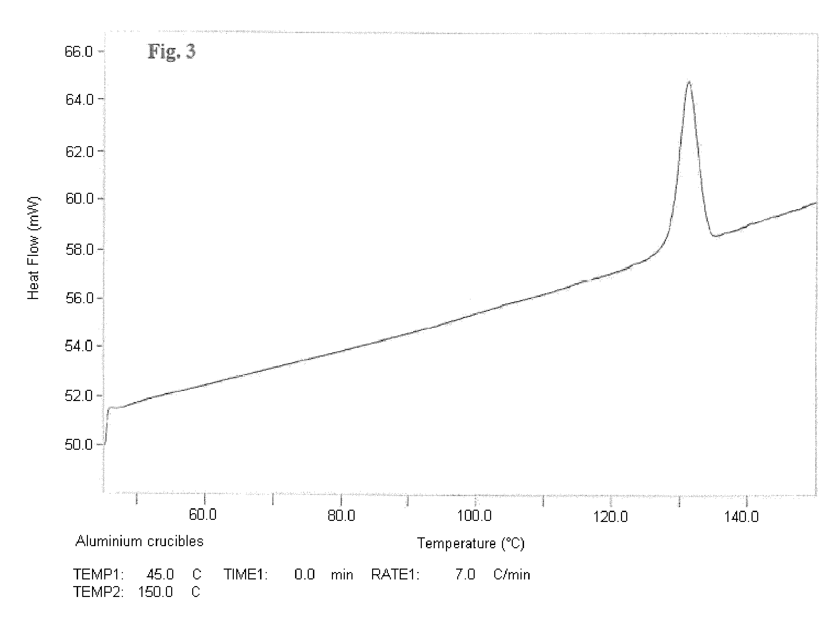

A first object of the invention is therefore a crystalline form of the compound of formula I, characterised by an endothermic transition between 20 and 30 J/g, at temperatures from 125°C to 135°C, determined by differential scanning calorimetry (DSC). A typical DSC scan is reported in Figure 3.

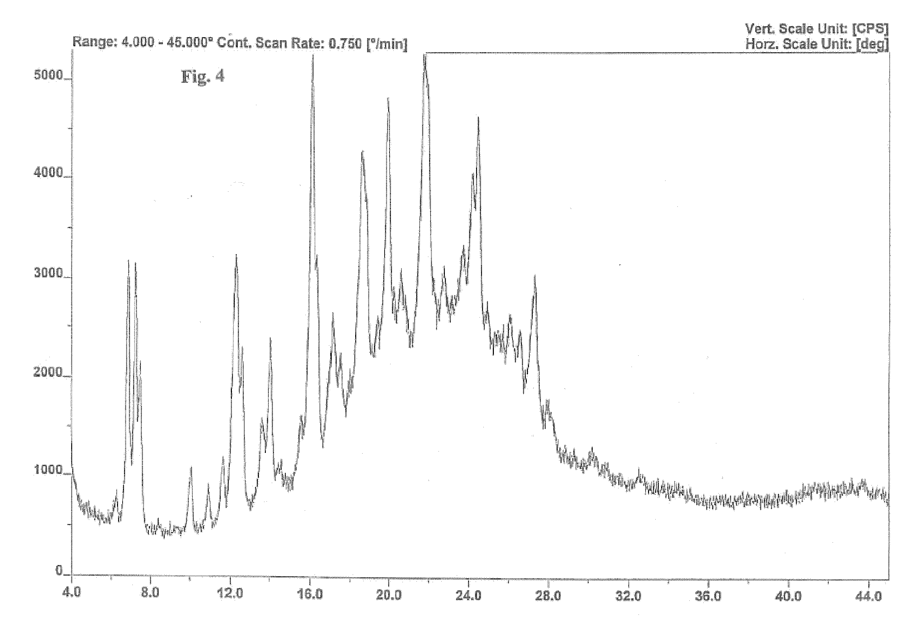

The crystalline form of the invention can also be characterised by other analysis techniques, such as X-ray diffractometry. For this purpose, an X-ray powder diffraction spectrum of the crystalline form according to the present invention is reported in Figure 4. The difference between the two crystalline forms is evident from a comparison of Figures 2 and 4: there is not the slightest overlap of the signals at the various angles of determination of the intensity of diffracted radiation.

The novel crystalline form can coexist with the known crystalline form or with the amorphous solid form already disclosed in MI2012A002009.

However, it is preferable for the known crystalline form not to be present, or to be present in minimal amounts, as it is not readily soluble.

The crystalline form of invention is preferably present to an extent exceeding 10% by weight, more preferably exceeding 40%, and even more preferably exceeding 80% by weight.

The novel crystalline form is obtained easily and efficiently by heating the amorphous solid form disclosed in Italian patent application MI2012A002009 to temperatures from 30°C to its melting point on a surface or in a non-solvent fluid. Said process can be performed in the absence or presence of an inert non-solvent liquid such as water or n-heptane. Depending on the time and temperature, mixed amorphous and crystalline forms can also be obtained, which form part of the invention. The formation of the novel crystalline form is generally promoted by operating at higher temperatures and for longer times.

The crystalline form of the invention is also easily obtained by cooling compound (I) in the molten state and maintaining it at temperatures below its melting point for times exceeding at least 20 minutes. In fact, excessively rapid cooling of the molten product leads to the production of the amorphous solid form as described in said Italian patent application. Said process can be performed in the absence or presence of an inert non-solvent liquid such as water or n-heptane. Depending on the time and temperature, mixed amorphous and crystalline forms can therefore also be obtained, which form part of the invention. The formation of the novel crystalline form is generally promoted by operating at higher temperatures and for longer times.

The crystalline form of the invention is also obtained by heating the known crystalline form, characterised by the X-ray spectrum shown in Figure 1 and by a DSC curve with endothermic enthalpy between 50 and 60 J/g, at temperatures from 120 and 130°C. This process can be performed in the absence or the presence of an inert non-solvent liquid such as water or n-octane.

The crystalline form of the invention is also easily obtained by completely removing the solvent from solutions of compound (I), provided that temperatures lower than the melting point are used. If this process is too fast, namely if it takes place in a period of between 20 minutes and a few hours, mixed amorphous and crystalline forms can be obtained, which also form part of the invention. Once again, the formation of the novel crystalline form is promoted by operating at higher temperatures and for longer times.

The non-solvents that can be used include water, methanol, heptane, octane and decane.

The solvents that can be used include alcohols such as ethanol, propanol, isopropanol, butanol, isobutanol, hexanol, 2-ethylhexanol, octanol and dodecanol; glycols such as propylene glycol; ketones such as acetone, methyl ethyl ketone, and methyl isobutyl ketone; esters such as ethyl acetate and butyl acetate; and aliphatic and aromatic hydrocarbons such as petrols, white spirit, benzene, toluene and xylene. In particular, 2-ethylhexanol is preferred.

Compound (I) can be synthesised by the techniques already known and described in US 4617390 and US 4724137 by reacting cyanuryl chloride with 2-ethylhexyl p-aminobenzoate in a solvent such as hydrocarbon fractions or xylene. The known crystalline form of compound (I) is obtained in said patents by recrystallisation from solvent.

The crystalline form of the invention can also be prepared from the solution of compound (I) as obtained at the end of the reaction described in example 1 of US 4617390, without proceeding to the subsequent recrystallisation steps but first removing the solvent and slowly cooling at a temperature below the melting point of the molten substance obtained, or gradually removing the solvent at temperatures below the melting point.

The compound of formula (I) can also be prepared by a novel process which constitutes a further object of the invention.

The novel process involves synthesis of a triazine ester intermediate of formula (II) wherein the alcohol fraction consists of one or more alcohols with a boiling point lower than that of the alcohol or alcohols obtainable from the alcohol fraction of the triazine compound of formula (III).

The compound of formula (III) is obtained subsequently by transesterification of the compound of formula (II) with an alcohol having a higher boiling point so as to remove, for example by distillation under vacuum, all the most volatile alcohol fractions, namely those with a lower boiling point. 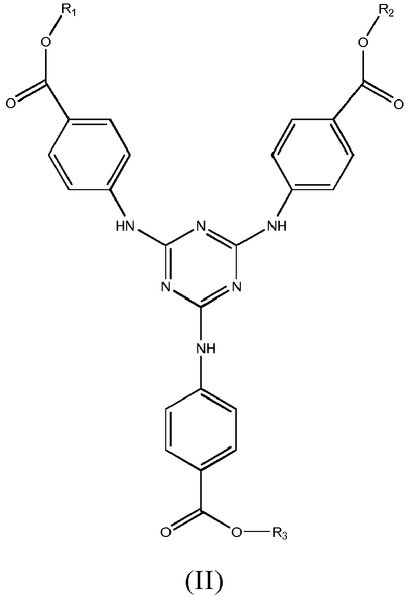 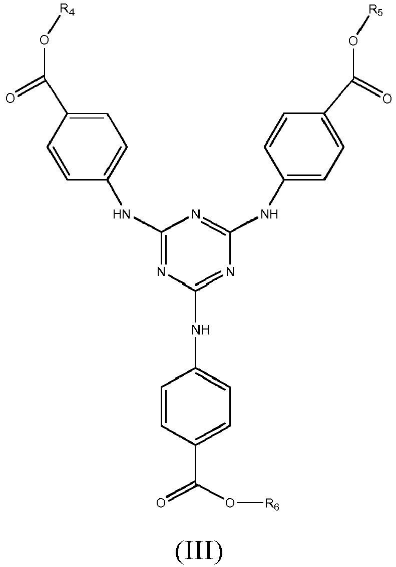 wherein R₁, R₂, R₃, R₄, R₅ and R₆ are optionally substituted C1-C22 alkyl, isoalkyl and aromatic groups, with the proviso that R₄, R₅ and R₆ cannot be the methyl group
and
wherein alcohols R₁OH, R₂OH and R₃OH have boiling points lower than those of alcohols R₄OH, R₅OH and R₆OH.
R₁, R₂, R₃, R₄, R₅ and R₆ are preferably alkyl or C1-C8 isoalkyl groups, and more preferably methyl, ethyl, propyl, isopropyl, octyl and 2-ethylhexyl groups
with the proviso that alcohols R₁OH, R₂OH and R₃OH have boiling points lower than those of alcohols R₄OH, R₅OH and R₆OH and that R₄OH, R₅OH and R₆OH cannot be methyl alcohol.

Even more preferably, R₁, R₂ and R₃ are, independently of one another, the methyl, ethyl, propyl and isopropyl groups, and more preferably, R₄, R₅ and R₆ are the 2-ethylhexyl group.

The process of the invention is summarised in the present scheme:
step (a) - preparation of the intermediate of formula (II) from a cyanuryl halide and a p-aminobenzoic acid ester derivative according to the processes disclosed in the above-mentioned US 4617390 and US 4724137;
step (b) - transesterification of the intermediate of formula (II) with one or more alcohols R₄OH, R₅OH and R₆OH, possibly in great excess, with complete elimination of R₁OH, R₂OH and R₃OH.
Step (a) can optionally be completed or perfected with purifications easily realised by neutralisation, filtration, washing and drying operations.
Step (b) can be performed with known techniques by reacting the compound of formula (II) with at least the stoichiometric amount of one or more alcohols of formula R₄OH, R₅OH and R₆OH, and completely removing alcohols R₁OH, R₂OH and R₃OH in the absence or presence of solvents and transesterification catalysts at temperatures from 0° to 250°C.
Step (b) can preferably be performed in the absence of a solvent and with an excess of alcohols R₄OH, R₅OH and R₆OH. Acids such as sulphuric acid, methanesulphonic acid and p-toluenesulphonic acid, or bases such as sodium hydroxide, Lewis acids, zirconates, stannates or titanates, for example tetraalkyl titanates such as tetrabutyl titanate and tetra(2-ethylhexyl)titanate, can be used as transesterification catalysts.
Step (b) can also be perfected with successive steps of elimination of the excess alcohols and any traces of catalyst, and washing, neutralisation, filtration and drying operations.

The process of the invention is preferably used for the preparation of the amorphous form and the crystalline form of the compound of formula (I), namely the compound of formula (III) wherein R₄, R₅ and R₆ are 2-ethylhexyl, through intermediate (II) wherein R₁, R₂ and R₃ are methyl and ethyl groups.

Operating by the process of the invention, it is preferable to end step (b) with a compound of formula (I) in molten form so that the amorphous form or the crystalline form of the invention can easily be obtained by cooling.

The crystalline form of the invention can be advantageously used in powder form or compacted in granules or flakes and used in plastics, coatings, detergents, cosmetic formulations, and in particular in sunscreens.

The novel crystalline form has greater resistance to storage at high environmental temperatures, such as 50°C, than the amorphous form. In particular, the particles of said form do not sinterise or agglomerate irreversibly at these temperatures.

Many cosmetic formulations consist of emulsions containing an oil phase. In particular, cosmetic formulations containing sunscreens such as the compound of formula (I) are generally of this type. The method most commonly used for their manufacture requires the sunscreen to be dissolved first in the oil phase, followed by production of the emulsion. The dissolution rate of the sunscreen and the temperature at which said dissolution takes place in reasonable times are therefore important economic factors.

The crystalline form of the invention can be dissolved easily in many emollients and polar oils typically used in the cosmetic field, especially in sunscreen formulations. Examples of said oils are (INCI Names): Hexyl Laurate, C12-13 Alkyl Lactate, PPG-3 Myristyl Ether, Propylene Glycol Monoisostearate, Di-C12-13 Alkyl Malate, C 12-13 Alkyl Octanoate, Cocoglycerides, Tridecyl Salicylate, Di-C12-13 Alkyl Tartrate, PEG-7 Hydrogenated Castor Oil, Dioctyl Adipate, Octyldodecanol, PPG-2 Myristyl Ether Propionate, Propylene Glycol Dicaprylate/ Dicaprate, Isopropyl PPG-2 Isodeceth-7-Carboxylate, PEG-7 Glyceryl Cocoate, Diisopropyl Adipate, Cetearyl Isononanoate, Coco Caprylate/Caprate, Dicaprylyl Maleate, Diethylhexyl Malate, Ethylhexyl Cocoate, Ethylhexyl Ethylhexanoate, Ethylhexyl Isostearate, Ethylhexyl Methoxycinnamate, Ethylhexyl Palmitate, Ethylhexyl Salicylate, C12-C15 Alkyl Benzoate, Caprylic/Capric Triglyceride, Isopropyl Myristate, Isopropyl Palmitate, Isopropyl Stearate, Ethylhexyl Stearate, Ethylhexyl Benzoate, Propylene Glycol Dicaprylate/Dicaprate.

The crystalline form of the invention can also be dissolved, optionally hot, in typical solvents, for example alcohols such as ethanol, propanol, isopropanol, butanol and 2-ethylhexanol, glycols such as propylene glycol, esters such as ethyl acetate and butyl acetate, ethers such as tetrahydrofuran, ketones such as acetone, methyl ethyl ketone and methyl isobutyl ketone, and aliphatic and aromatic hydrocarbons such as toluene and xylenes.

The crystalline form of the invention can also be dissolved in other UVA and UVB sunscreens in the liquid state.

Within the narrower solubility ranges applicable in various cases, the novel crystalline form of the present invention can also be used in less polar oils, such as liquid paraffin and dicaprylyl ether.

In particular, the crystalline form of the invention can be advantageously introduced into cosmetic formulas, either as the only sunscreen or in combination with other known sunscreens, instead of the forms obtained by known processes.

These formulations constitute a second object of the invention. Said formulations will preferably contain one or more conventional UVA and UVB sunscreens such as those listed in Annex VII to the European Cosmetics Directive (76/768/EEC). Even more preferably, the formulations may contain, in addition to the crystalline form of the invention, one or more sunscreens selected from 2-ethylhexyl p-methoxycinnamate, 2-hydroxy-4-methoxybenzophenone, 2-hydroxy-4-methoxybenzophenone-5-sulphonic acid, 3-(4'-methylbenzylidene)-d,l-camphor, diethylhexyl butamido triazone, 4-(tert-butyl)-4'-methoxy-dibenzoylmethane, 2-cyano-3,3-diphenylacrylic acid 2-ethylhexyl ester, bis-ethylhexyloxyphenol-methoxyphenyl-triazine, methylene-bis-benzotriazolyl-tetramethylbutylphenol, benzoic acid 2-(4-diethylamino-2-hydroxybenzoyl)-hexyl ester, titanium dioxide and zinc oxide.

The examples below illustrate the invention in greater detail.

All the DSC characterisations were performed under nitrogen and at a heating rate of 7°C/min. The X-ray powder diffraction characterisations were determined with a Thermo Scientific, X'TRA 132 X-ray diffractometer with source: Cu X-ray tube - emission line: X-ray.

### Example 1 - production of the amorphous form of compound of formula (I)

100 g of Uvasorb ET (3V Sigma Spa) in the form of a white crystalline powder (sample 1), corresponding to the compound of formula (I), was melted at 150°C to obtain a slightly viscous liquid, and poured rapidly onto a flat PTFE tray at 25°C. Within 5 minutes a fragile vitreous mass formed, which was ground and sieved through a 200 micron mesh sieve to obtain a white powder (sample 2).

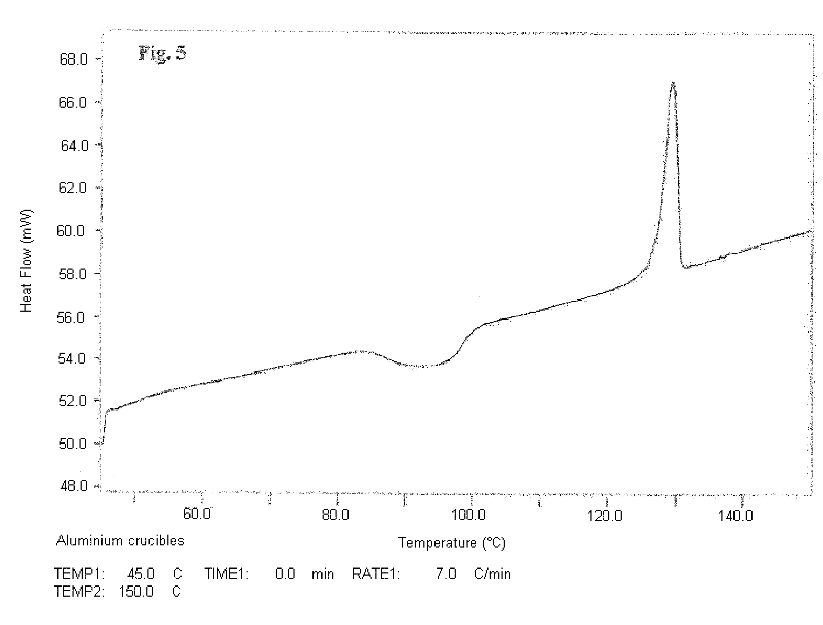

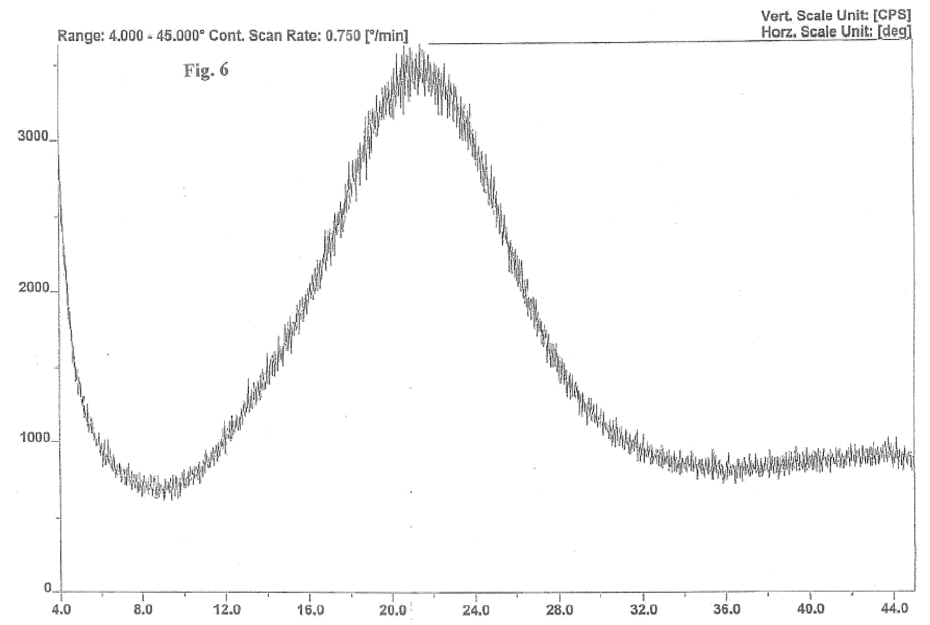

Samples 1 and 2 were characterised by the differential scanning calorimetry (DSC) technique. Sample 1 exhibited a thermogram without any thermal transition up to 120°C, and a very intense endothermic transition with a peak at 126°C, as shown in Figure 1. The energy contribution of this transition is 55 J/g of substance. The X-ray powder diffraction spectrum of sample 1 is reported in Figure 2. The amorphous solid sample 2 exhibited a first exothermic transition at temperatures from 50 to 90°C and subsequently at 129°C the endothermic transition of 28 J/g corresponding to the melting of the crystalline form of the present invention that formed at lower temperatures during the exothermic event. The DSC curve of amorphous sample 2 is reported in Figure 5. The X-ray powder diffraction spectrum of this sample is reported in Figure 6. The absence of signals typical of samples without an orderly crystalline structure is clearly seen in said figure. Sample 2 was stored for 12 months at room temperature and subjected to a second DSC measurement. Its appearance was still that of a white powder. The DSC curve of this sample, aged for 12 months, still presented the exothermic peak between 50 and 90°C, and was identical to the curve obtained a year earlier.

Sample 2 is therefore an amorphous, solid, stable form of the compound of formula (I).

### Example 2 - production of the amorphous form of compound of formula (I)

Example 1 was repeated using 100 g of Uvinul T 150 (Basf) in the form of a white crystalline powder (sample 3) to obtain the corresponding amorphous solid form (sample 4).

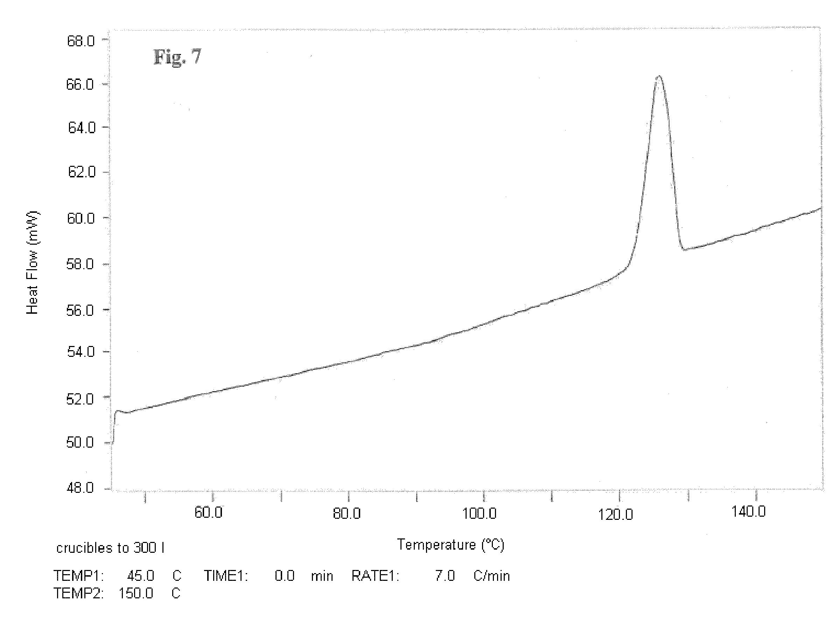

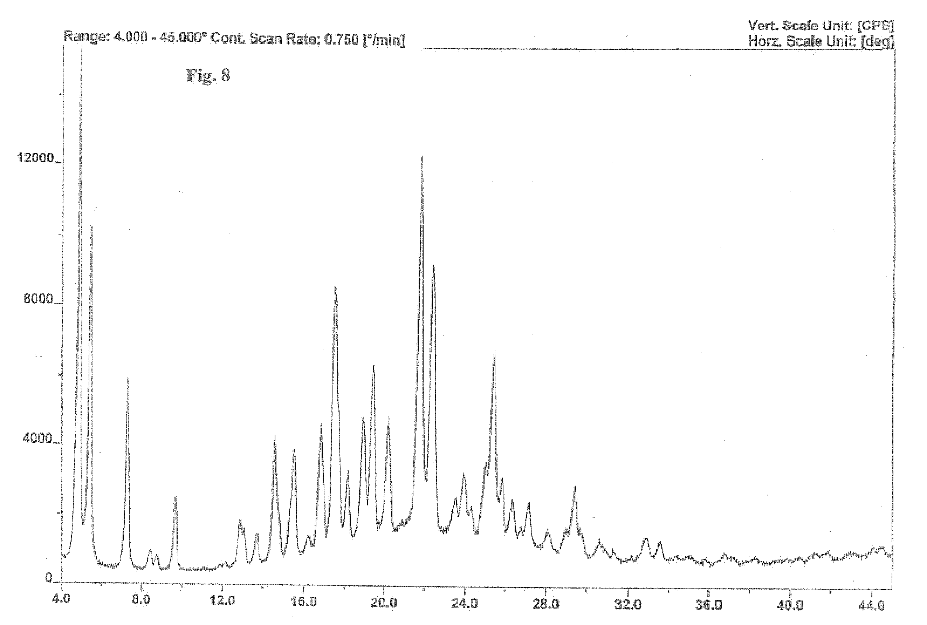

The X-ray powder spectrum of sample 3 is reported in Figure 8, and is identical to that of Figure 2 relating to sample 1. The DSC thermogram of sample 3, reported in Figure 7, is also similar to that of sample 1 in Figure 1, relating to the known crystalline form.

Once again, the DSC technique proved that sample 4 was a stable amorphous form of the compound of formula (I).

### Example 3 - crystalline form of the invention, obtained from the molten product

30 g of sample 1 (Uvasorb ET - 3V Sigma Spa) was melted in an oven at 150°C and then treated at 121°C for 4 hours. The sample was then cooled, ground and sieved through a 200 micron mesh sieve. 25 g of sample 5 in powder form, with a DSC thermogram corresponding to the one shown in Figure 3 and the X-ray diffraction spectrum reported in Figure 4, was obtained. Figure 3 shows a single endothermic melting transition of between 26 and 30 J/g. The X-ray spectrum in Figure 4 clearly proves that sample 5 consists of a crystalline form different from the one already known, the spectra of which are reported in Figures 2 and 8.

### Example 4 - crystalline form of the invention, obtained from the amorphous form

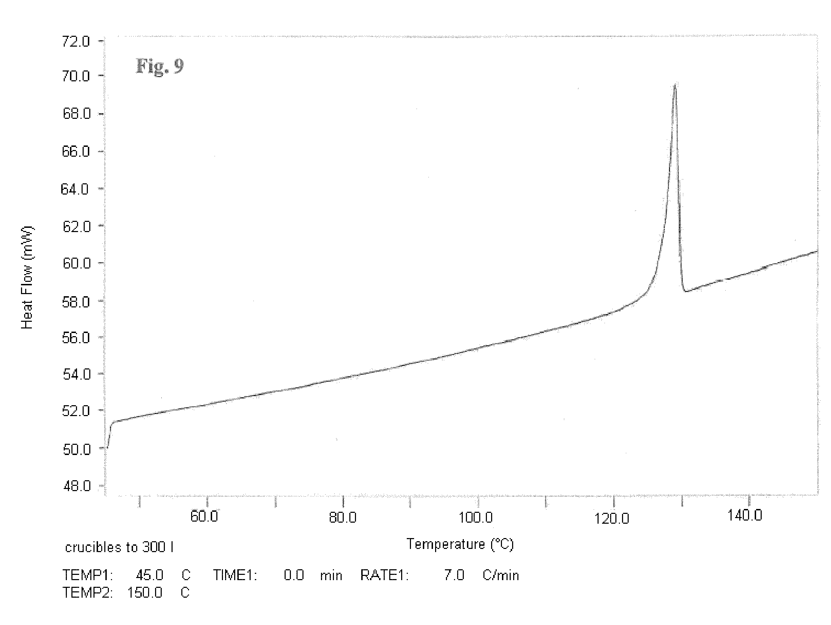

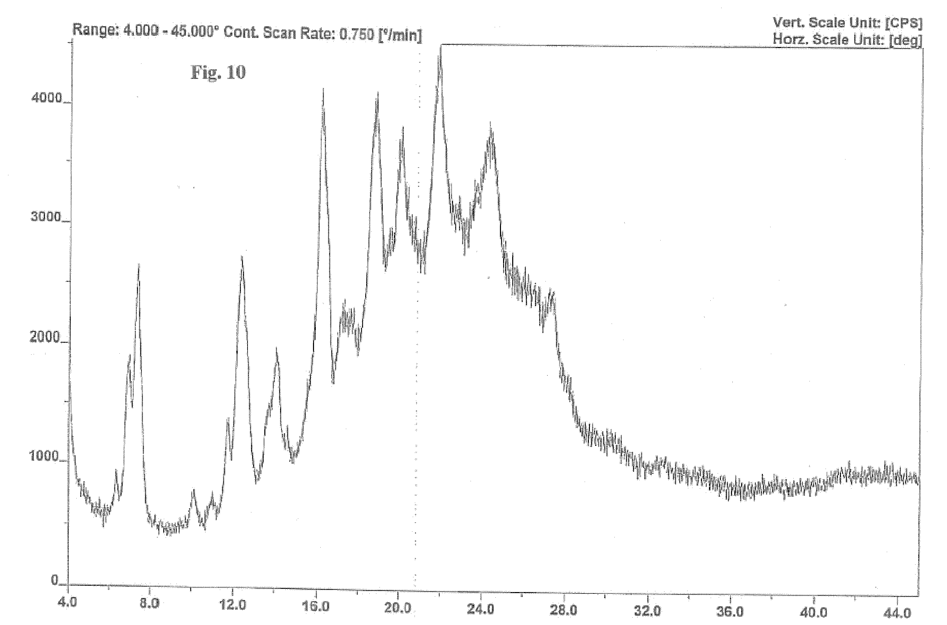

30 g of sample 2 (amorphous) was treated at 90°C for 150 minutes. The sample was then cooled, ground and sieved through a 200 micron mesh sieve. 25 g of sample 6 in the novel crystalline form, with the DSC thermogram reported in Figure 9 and the X-ray diffraction spectrum reported in Figure 10, was obtained.

### Example 5 - crystalline form of the invention, obtained from the molten product

Example 3 of the present invention was repeated using 100 g of compound (I) in accordance with the experimental conditions described in example 1 of US 4617390. The solvent was removed from the final reaction mixture at 150°C. The crystalline form of the invention was obtained by cooling the molten mass and subsequent treatment in an oven on a surface at 121°C for 4 hours, then ground and sieved through a 200 micron mesh sieve to obtain sample 7.

Once again, the DSC technique proved that sample 7 was the novel crystalline form of the compound of formula (I).

### Example 6 - crystalline form of the invention

50 g of 1,3,5-Tris-(p-ethoxycarbonyl aniline)-s-triazine (CAS 96474-94-1), obtained according to example 2 of US 4617390 and corresponding to step (a) of the process of the invention, was dispersed in 250 g of 2-ethylhexanol containing 0.5 g of tetra-n-butyl-ortho-titanate and transesterified at 150°C, completely removing ethanol. The solution of the compound of formula (I) in 2-ethylhexanol was washed twice with water, clarified and then heated to 140°C under vacuum to remove all the residual 2-ethylhexanol.

The molten liquid product was flaked in 10 minutes on a PTFE tray at 20°C to obtain transparent vitreous flakes corresponding to the amorphous form of the compound of formula (I). The flakes were ground and sieved through a 200 micron mesh sieve to obtain powdered sample 8. Sample 8 was treated in an oven at 121°C for 6 hours, and then cooled to room temperature, ground and sieved through a 200 micron mesh sieve until 70 g of powdered sample 9, corresponding to the crystalline form of the invention, was obtained.

Sample 8 was subjected to DSC, and exhibited an exothermic crystallisation peak between 60 and 90°C and a subsequent endothermic peak of comparable extent at 129°C. The DSC thermogram of sample 8 is typical of the amorphous form of the compound of formula (I). Sample 9 was similarly subjected to DSC analysis, and exhibited a thermogram corresponding to that of the crystalline form of the invention.

### Application example 7

### Description of dissolution rate test.

4.0 g of powder was dispersed under stirring in 96.0 g of caprylic/capric triglyceride solvent at 25°C.

The total dissolution time was measured, with visual inspection, noting the time of complete disappearance of any heterogeneity in the form of particles or opalescence in the solution.

### Results.

| | |
|---|---|
| Sample 1 | dissolution time over 90 minutes |
| Sample 2 | 15 minutes |
| Sample 3 | over 90 minutes |
| Sample 4 | 15 minutes |
| Sample 5 | 12 minutes |
| Sample 6 | 12 minutes |

### Application example 8

### Description of dissolution rate test.

4.0 g of powder was dispersed under stirring in 96.0 g of ethylhexyl stearate solvent at 25°C.

The total dissolution time was measured, with visual inspection, noting the time of complete disappearance of any heterogeneity in the form of particles or opalescence in the solution.

### Results.

| | |
|---|---|
| Sample 5 | dissolution time 35 minutes |
| Sample 1 | over 420 minutes (opaque white dispersion) |

### Application example 9

### Description of dissolution rate test.

4.0 g of powder was dispersed under stirring in 96.0 g of octyl dodecanol solvent at 25°C.

The total dissolution time was measured, with visual inspection, noting the time of complete disappearance of any heterogeneity in the form of particles or opalescence in the solution.

### Results.

| | |
|---|---|
| Sample 6 | dissolution time 80 minutes |
| Sample 3 | over 420 minutes (insoluble - opaque white dispersion) |

### Application example 10

The powdered crystalline sample 5 according to the present invention was used to prepare two cosmetic formulas of oil-in-water emulsion (cosmetic formula 1 and cosmetic formula 2). The sun protection factor was measured *in vitro* (SPF *in vitro*) on said two formulas with a Labsphere UV-2000S instrument, in the UV-visible zone from 290 to 400 nm. For the experimental measurement of the SPF the cosmetic formula was applied to Transpore tape (3M Inc.) at the concentration of 2.0 mg/cm². 3 tapes were prepared for each formula, 12 readings being taken per tape, and readings with covariance >10% above the average were rejected.

The following mean values were obtained:

| | |
|---|---|
| Cosmetic formula 1 | SPF *in vitro = 5.0* |
| Cosmetic formula 2 | SPF *in vitro =* 14.4 |

### Cosmetic formula 1

Preparation: phase I and phase II are heated separately, under stirring, at 70-75°C, until the ingredients are completely solubilised. Phase II is added to phase I, maintaining the same temperature and emulsifying with a Silverson homogeniser at 3000 rpm. After the addition of phase III, the preparation is cooled to 40°C. After the addition of phase IV, the product obtained is discharged.

| **Phase** | **Ingredient** | **INCI name** | **% (w/w**) |
|---|---|---|---|
| **I** | Cremophor GS32 | Polyglyceryl-3 Distearate | 3 |
| | Crodet S40 | PEG-40 Stearate | 0.3 |
| | Lanette O | Cetearyl Alcohol | 2 |
| | Cutina GMS | Glyceryl Stearate | 1 |
| | Cetiol OE | Dicaprylyl Ether | 7.5 |
| | Ceraphyl 230 | Diisopropyl Adipate | 10.5 |
| | Sample 5 | Ethylhexyl Triazone | 3 |
| **II** | Demineralised water | Water | Up to 100 |
| | EDTA | EDTA | 0.1 |
| | Synthalen K | Carbomer | 0.15 |
| | Propylene glycol | Propylene Glycol | 3 |
| **III** | Triethanolamine | TEA | 0.2 |
| **IV** | Microcare PM5 | Methyl paraben, Ethyl paraben, Propyl paraben, Butyl paraben, Isobutyl paraben, 2-phenoxyethanol | 0.5 |

### Cosmetic formula 2

Preparation: phase I and phase II are heated separately, under stirring, at 70-75°C, until the ingredients are completely solubilised. Phase II is added to phase I, maintaining the same temperature and emulsifying with a Silverson homogeniser at 3000 rpm. After the addition of phase III, the preparation is cooled to 40°C. After the addition of phase IV, the product obtained is discharged.

| **Phase** | **Ingredient** | **INCI name** | **% (w/w)** |
|---|---|---|---|
| **I** | Simusol 165 | Glyceryl Stearate (and) PEG-100 Stearate | 2 |
| | Lanette O | Cetearyl Alcohol | 0,5 |
| | Cetiol AB | C12-15 Alkyl Benzoate | 18 |
| | Sample 5 | Ethylhexyl Triazone | 2 |
| | Eusolex 9020 | Butyl Methoxydibenzoylmethane | 5 |
| | Eusolex OCR | Octocrylene | 5 |
| | Ueusolex 2292 | Ethylhexyl Methoxycinnamate | 0,1 |
| **II** | Demineralised water | Aqua | Up to 100 |
| | Glycerin | Glycerin | 3 |
| | Synthalen K | Carbomer | 0,15 |
| **III** | Triethanolamine | TEA | 0,2 |
| **IV** | Microcare PM5 | Methyl paraben, Ethyl paraben, Propyl paraben, Butyl paraben, Isobutyl paraben, 2-phenoxyethanol | 0,5 |

### Application example 11 - resistance to high storage temperatures

20 g of sample 8 (amorphous form) was placed on a PTFE tray with a 10 cm diameter and treated in an oven at 50°C for 48 hours.

Similarly, 20 g of sample 9 (crystalline form of the present invention) was placed on a PTFE tray with a 10 cm diameter and treated in an oven at 50°C for 48 hours.

At the end of the test the powder of sample 8 was compact and sinterised, whereas sample 9 was almost unchanged.

This experiment demonstrates that the crystalline form according to the present invention is more resistant to high temperatures and storage than the corresponding amorphous form.

## Claims

1. Crystalline form of the compound of formula (I) **characterised by** an endothermic transition at temperatures from 125°C to 135°C ranging from 20 to 30 J/g as determined by differential scanning calorimetry (DSC).

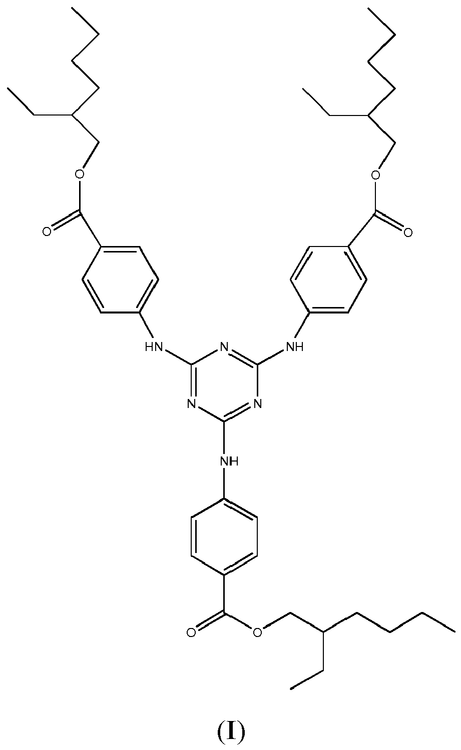

2. Crystalline form according to claim 1 **characterised by** the X-ray powder diffraction spectrum reported in figure 4 and figure 10.

3. Process for the production of the crystalline form of claims 1-2 by cooling the melted compound (I) at temperatures below the melting point on a surface or in a non-solvent fluid.

4. Process for the production of the crystalline form of claims 1-2 by thermal treatment of the amorphous form of the compound of formula (I) at temperatures ranging from 30°C to the melting point on a surface or in a non-solvent fluid.

5. Process for the production of the crystalline form of claims 1-2 by thermal treatment of the known crystalline form of the compound of formula (I) at temperatures ranging from 120°C to the melting point on a surface or in a non-solvent fluid.

6. Process according to any one of claims 3, 4, 5 wherein the fluid is selected from nitrogen, air or a non-solvent selected from water, heptane or octane.

7. Process for the production of the crystalline form of claims 1-2 by removing the solvent from a solution of the compound of formula (I) at temperatures below the melting point.

8. Process according to claim 7 wherein the solvent is a C4-C12 aliphatic alcohol.

9. Process according to claim 8 wherein the solvent is an octanol, particularly 2-ethylhexanol.

10. Process for the production of the crystalline form of claims 1-2 which comprises:
a) synthesis of a triazine intermediate of formula (II) 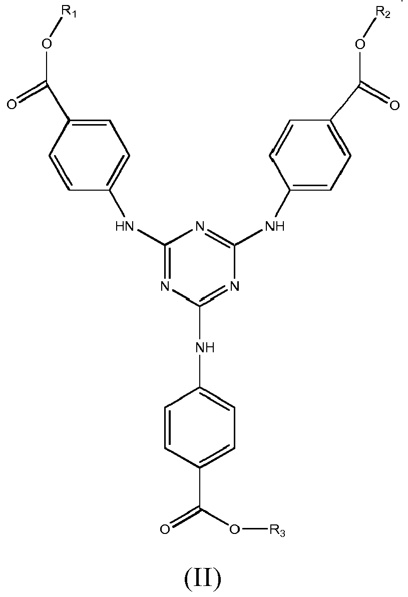 wherein R₁, R₂, R₃ are optionally substituted C1-C22 alkyl, isoalkyl and aromatic groups;
b) transesterification of the compound of formula (II) with alcohols of formula R₄OH, R₅OH, R₆OH to give a compound of formula III 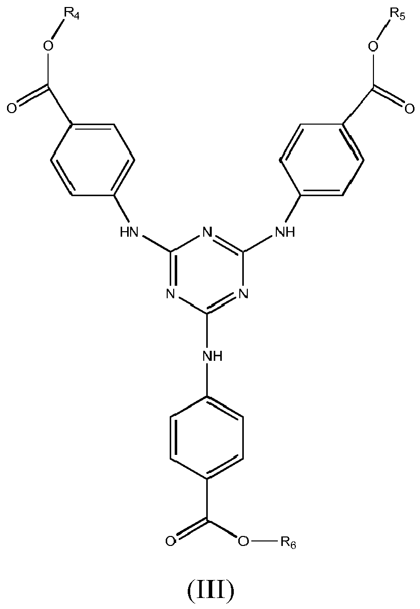 wherein R₄, R₅, R₆ are optionally substituted C1-C22 alkyl, isoalkyl and aromatic groups, with the proviso that alcohols R₁OH, R₂OH, R₃OH have boiling points below those of alcohols R₄OH, R₅OH, R₆OH;
c) removal of the most volatile alcoholic fractions.

11. Use of the solid form of claims 1-2 in the form of powder, granules or flakes in plastics, coatings, detergents, cosmetic formulations and particularly in sunscreens.

12. Use of the solid form according to claims 1-2 in the form of powder, granules or flakes in combination with other known UVA and UVB sunscreens.

13. Use according to claims 11 and 12 wherein the UVA and UVB sunscreens are selected from 2-ethylhexyl p-methoxycinnamate, 2-hydroxy-4-methoxybenzophenone, 2-hydroxy-4-methoxybenzophenone-5-sulphonic acid, 3-(4'-methylbenzylidene)-d,l-camphor, diethylhexyl butamido triazone, 4-(tert-butyl)-4'-methoxy-dibenzoylmethane, 2-cyano-3,3-diphenylacrylic 2-ethylhexyl ester, bis-ethylhexyloxyphenol-methoxyphenyl-triazine, methylene-bis-benzotriazolyl-tetramethylbutylphenol, benzoic acid 2-(4-diethylamino-2-hydroxybenzoyl)-hexyl ester, titanium dioxide, zinc oxide.

14. Cosmetic formulations comprising the solid form of claims 1-2, optionally in mixtures with other UVA or UVB filters.

15. Cosmetic formulations according to claim 14 wherein additional UVA and UVB filters are selected from 2-ethylhexyl p-methoxycinnamate, 2-hydroxy-4-methoxybenzophenone, 2-hydroxy-4-methoxybenzophenone-5-sulphonic acid, 3-(4'-methylbenzylidene)-d,l-camphor, diethylhexyl butamido triazone, 4-(tert-butyl)-4'-methoxy-dibenzoylmethane, 2-cyano-3,3-diphenylacrylic 2-ethylhexyl ester, bis-ethylhexyloxyphenol-methoxyphenyl-triazine, methylene-bis-benzotriazolyl-tetramethylbutylphenol, benzoic acid 2-(4-diethylamino-2-hydroxybenzoyl)-hexyl ester, titanium dioxide, zinc oxide.

16. Method for the preparation of cosmetic formulations which comprises solubilisation of the solid form of claims 1-2 in a cosmetic oil.
